# FASCICULE DE BREVET EUROPEEN

(11) **EP 3 713 665 B1**
(45) Date de publication et mention de la délivrance du brevet: **04.08.2021**
(21) Numéro de dépôt: 18803691.7
(22) Date de dépôt: 20.11.2018
(51) Int. Cl.: A23P 10/30, B01J 13/14, A23P 30/25, A61K 8/04, A61K 8/11, A61K 8/20, A61K 9/50, A61Q 19/00, A61K 35/12

(54) **PROCÉDE DE FABRICATION DE CAPSULES FORMÉES D'UNE ENVELOPPE EXTERNE D'HYDROGEL RÉTICULÉ ENTOURANT UN NOYAU CENTRAL**
VERFAHREN ZUR HERSTELLUNG VON KAPSELN AUS EINER ÄUSSEREN HÜLLE AUS VERNETZTEM HYDROGEL, DIE EINEN ZENTRALEN KERN UMGIBT
PROCESS FOR PRODUCING CAPSULES MADE OF AN EXTERNAL SHELL OF CROSSLINKED HYDROGEL SURROUNDING A CENTRAL CORE

(30) Priorité: 21.11.2017 FR 1761018
(43) Date de publication de la demande: 30.09.2020
(73) Titulaire: Université de Bordeaux, 33000 Bordeaux (FR); Centre National de la Recherche Scientifique, 75016 Paris (FR); Institut d'Optique Theorique et Appliquée, 91120 Palaiseau CEDEX (FR)
(72) Inventeur: FEYEUX, Maxime, 33400 Talence (FR); ALESSANDRI, Kevin, 33000 Bordeaux (FR); NASSOY, Pierre, 33000 Bordeaux (FR)
(74) Mandataire: Cabinet Becker et Associés
(86) Numéro de dépôt international: PCT/EP2018/081913
(87) Numéro de publication internationale: WO 2019/101734

(56) Documents cités:
- JP-A- S63 236 534
- US-A1- 2005 158 395
- US-A1- 2011 008 293
- US-A1- 2013 078 308
- US-B1- 6 377 387
- IRMANIDA BATUBARA ET AL: "Leydig Cells Encapsulation with Alginate-Chitosan: Optimization of Microcapsule Formation", JOURNAL OF ENCAPSULATION AND ADSORPTION SCIENCES, vol. 02, no. 02, 1 juin 2012 (2012-06-01), pages 15-20, XP055478042, ISSN: 2161-4865, DOI: 10.4236/jeas.2012.22003

## Description

La présente invention concerne un procédé de fabrication de capsules formées d'une enveloppe externe d'hydrogel réticulé entourant un noyau central liquide. Plus précisément, l'invention a trait à un procédé de fabrication permettant d'encapsuler une composition d'intérêt, pour former des capsules dans lesquelles les propriétés de ladite composition d'intérêt sont préservées.

Les procédés d'encapsulation se sont développés ces dernières années afin de fabriquer des capsules constituées d'une enveloppe externe renfermant une composition centrale, le plus souvent liquide. De telles capsules sont notamment utilisées dans le domaine pharmaceutique et cosmétique. La composition centrale comprend alors un principe actif protégé des agressions externes et dont le relargage peut être mieux contrôlé.

Généralement, les capsules sont obtenues en coextrudant de manière concentrique une solution d'hydrogel, destinée après réticulation à former l'enveloppe externe, avec une composition d'intérêt à encapsuler. Au sortir de l'extrudeuse, on obtient des gouttes mixtes, composées d'une couche de solution d'hydrogel entourant une goutte de la composition d'intérêt. Les gouttes mixtes tombent alors dans un bain comprenant des agents de réticulation, dans lequel la réticulation de la couche d'hydrogel a lieu.

Cependant, on observe souvent une déstructuration des gouttes mixtes lors de leur chute depuis l'extrudeuse jusqu'au bain de réticulation et/ou lors de l'impact des gouttes mixtes avec le bain de réticulation. La couche externe d'hydrogel se rompt partiellement ou totalement sous la violence de l'impact, ce qui entraîne un mélange partiel de la goutte de composition d'intérêt avec la couche liquide d'hydrogel avant réticulation, voire l'expulsion de la goutte de composition d'intérêt vers l'extérieur de la goutte mixte au cours de la réticulation de l'hydrogel. Aussi, les capsules récupérées ne sont pas de forme régulière, ni de dimensions et/ou de topologie contrôlée.

En conséquence, il est souvent nécessaire d'ajouter un tensioactif dans la solution d'hydrogel, afin d'éviter que les gouttes mixtes n'éclatent lors de leur impact avec le bain de réticulation. Les tensioactifs peuvent entrer en contact avec le contenu de la capsule et en altérer les propriétés. Ainsi, lorsque les capsules renferment des principes actifs ou des cellules, cela peut avoir des conséquences négatives importantes, et notamment diminuer l'efficacité du principe actif ou entraîner une mort cellulaire. De même, lorsque les capsules renferment une composition agroalimentaire, les propriétés organoleptiques de ladite composition peuvent être modifiées par l'interaction avec le tensioactif.

Par ailleurs, la concentration d'agents de réticulation est nécessairement élevée pour figer la structure de la goutte à l'impact. En conséquence, les agents de réticulation traversent la couche de solution d'hydrogel au cours de la réticulation et pénètrent à l'intérieur de la capsule. Au même titre que l'interaction avec les tensioactifs, le contact de l'agent de réticulation avec l'intérieur de la capsule peut altérer les propriétés de son contenu.

Ces agents de réticulation et tensioactifs peuvent par ailleurs être irritants pour le corps humain. Il est donc parfois nécessaire, en fonction notamment de la destination des capsules, de procéder à des étapes de rinçage poussées, pour éliminer toute trace d'agent de réticulation et/ou de tensioactif à la surface et/ou au sein des capsules. Ces étapes de rinçage diminuent significativement le rendement et augmentent le coût de production de telles capsules.

Il existe donc un besoin en un procédé de fabrication de capsules d'hydrogel renfermant une composition d'intérêt, notamment une composition liquide, qui permette d'obtenir des capsules de forme et de structure contrôlées, sans coût excessif, à haut débit. Le document US 6 377 387 B1 concerne des procédés de production de grandes quantités de gouttelettes sensiblement monodispersées à utiliser dans des affichages électrophorétiques à base de capsules. Le document JP S63 236534 A concerne la production en série de capsules. Le document US 2005/158395 A1 concerne une méthode d'encapsulation de capsules à usage médical.

### Résumé de l'invention

En travaillant sur ces problématiques, les inventeurs ont découvert qu'il est possible de réticuler au moins partiellement la couche d'hydrogel autour de la goutte de composition d'intérêt avant que les capsules n'impactent le bain de récupération dans lequel elles sont réceptionnées. Une telle pré-réticulation de l'hydrogel autour de la goutte de composition d'intérêt prévient les risques de déstructuration des capsules. En outre, les risques de contact du contenu de la capsule avec un agent de réticulation et/ou un éventuel tensioactif sont également réduits. Pour permettre une telle réticulation précoce, les inventeurs ont développé un procédé dans lequel les gouttes mixtes, obtenues classiquement par coextrusion d'une solution d'hydrogel et de la composition d'intérêt, traversent un aérosol formé de microparticules (liquides ou solides) comprenant un agent de réticulation. Les microparticules viennent se coalescer à la surface des gouttes mixtes au cours de leur chute à travers ledit aérosol, et permettent la réticulation au moins partielle de l'hydrogel. Avantageusement, la couche la plus externe de l'hydrogel est réticulée, formant une coque rigide ou semi-rigide autour de la goutte de composition d'intérêt. Une pulvérisation ou brumisation par jet favoriserait à l'inverse la pénétration des microparticules d'agent de réticulation à l'intérieur des gouttes mixtes, provoquant ainsi un mélange de la couche externe d'hydrogel avec la goutte de composition d'intérêt formant le cœur de la goutte mixte, de nature à altérer la structuration de la goutte mixte. Contrairement à une pulvérisation par jet, l'utilisation d'une brume, ou aérosol, dans laquelle les microparticules sont en suspension dans l'air, favorise le dépôt d'une fine couche d'agent de réticulation tout autour des gouttes mixtes, provoquant une réticulation au moins partielle de la surface de la couche d'hydrogel. Un tel procédé permet avantageusement de s'affranchir de l'utilisation de tensio-actif, ce qui peut être particulièrement intéressant pour la réalisation de capsules contenant des cellules. Au besoin, si la réticulation n'est pas suffisante, il est possible de la poursuivre dans un bain de réticulation. Selon l'invention, la couche pré-réticulée d'hydrogel protège les capsules au moment de l'impact avec le liquide dans lequel elles sont réceptionnées après leur chute. Par ailleurs, en cas d'immersion de ces capsules dans un bain de réticulation, la présence de la couche pré-réticulée, même très fine, suffit à protéger la structure de la capsule et prévient sa déformation. Les temps d'immersion dans d'éventuels bains de réticulation peuvent en outre être réduits et les étapes de rinçage ultérieures également.

L'invention a donc pour objet un procédé de fabrication d'une pluralité de capsules comprenant chacune une enveloppe externe d'hydrogel réticulée entourant un noyau central, selon lequel une solution d'hydrogel et une composition d'intérêt aqueuse, destinée à former le noyau central, sont coextrudées de manière concentrique pour former des gouttes mixtes comprenant chacune une couche de solution d'hydrogel entourant une goutte de composition d'intérêt, caractérisé en ce que l'étape de coextrusion est réalisée au-dessus d'un aérosol de réticulation afin que les gouttes mixtes traversent ledit aérosol de réticulation, de sorte que la couche de solution d'hydrogel se réticule au moins partiellement autour de la goutte de composition d'intérêt au contact dudit aérosol.

Des capsules, susceptibles d'être obtenues par un tel procédé, dans lequel la surface externe de la couche d'hydrogel porte la trace de la mise en contact avec l'aérosol de réticulation sous forme d'irrégularités de surface causées par les impacts. De telles capsules se caractérisent donc par une couche externe d'hydrogel réticulé entourant un noyau central constitué d'une goutte de composition d'intérêt, dans lesquelles la surface externe de la couche d'hydrogel est alvéolée, c'est-à-dire recouverte d'alvéoles.

Des capsules telles que décrites ci-dessus peuvent être telles que le noyau central comprend des cellules présentant une surmortalité causée par l'encapsulation inférieure à 20%, préférentiellement inférieure à 10%.

### Description détaillée

Le procédé selon l'invention se caractérise par une étape de réticulation au moins partielle d'une couche d'hydrogel autour d'un noyau central au moyen d'un aérosol de réticulation, dont les microparticules viennent se coalescer à la surface de la couche d'hydrogel.

Plus précisément, les gouttes mixtes formées au sortir de l'extrudeuse sont projetées par le flux d'extrusion en direction d'une surface de réception et traversent l'aérosol disposé entre la sortie d'extrudeuse et la surface de réception.

La surface de réception s'entend de l'élément sur ou dans lequel les capsules tombent après avoir traversé l'aérosol de réticulation. Dans un mode de réalisation, la surface de réception consiste en un bain de récupération, comprenant éventuellement une solution de réticulation.

Selon l'invention tout procédé d'extrusion permettant de coextruder de manière concentrique une solution d'hydrogel et une composition d'intérêt peut être utilisé. Notamment, il est possible de réaliser les gouttes mixtes en adaptant la méthode et le dispositif microfluidique décrits dans Alessandri et al., (PNAS, September 10, 2013 vol. 110 no.37 14843-14848 ; Lab on a Chip, 2016, vol. 16, no. 9, p. 1593-1604) ou dans Onoe et al., (Nat Material 2013, 12(6):584-90), de manière à ce que l'ensemble des solutions soit coextrudé au-dessus d'un aérosol de réticulation. Par exemple, le procédé selon l'invention est mis en œuvre au moyen d'un dispositif d'extrusion à double ou triple enveloppes concentriques tel que décrit dans le brevet FR2986165.

Selon l'invention, on entend par « gouttes mixtes » les gouttes obtenues au sortir d'une extrudeuse au moyen de laquelle une solution d'hydrogel périphérique et une composition d'intérêt centrale ont été coextrudées de manière concentrique. Les gouttes mixtes sont donc composées d'une couche de solution d'hydrogel non réticulé entourant une goutte de composition d'intérêt. Par « goutte de composition d'intérêt », on entend un petit volume de la composition d'intérêt, qui peut être liquide, une solution, un colloïde ou une suspension, notamment sous forme de gel, de crème, etc. D'une manière générale, la composition d'intérêt est avantageusement aqueuse. Il peut ainsi s'agir d'une composition aqueuse sous forme liquide, de gel, de crème, etc. Dans le cas d'une composition d'intérêt sous forme de gel, ledit gel est avantageusement différent de l'hydrogel utilisé pour former la couche externe des gouttes mixtes. Préférentiellement, la composition d'intérêt ne comprend pas d'alginate, et plus généralement ne comprend pas de composés réticulables.

A l'inverse, on entend par « capsules » les produits issus des gouttes mixtes, après réticulation au moins partielle de la couche d'hydrogel. Les capsules sont donc composées d'une enveloppe externe d'hydrogel au moins partiellement rigidifiée par réticulation autour de la goutte de composition d'intérêt.

Les gouttes mixtes comme les capsules présentent avantageusement une forme sphérique.

Dans le contexte de l'invention, « l'enveloppe externe d'hydrogel réticulé » désigne une structure tridimensionnelle formée à partir d'une matrice de chaînes de polymères gonflée par un liquide, et préférentiellement de l'eau. Une telle enveloppe externe d'hydrogel réticulé est obtenue par réticulation d'une solution d'hydrogel. Avantageusement, le ou les polymères de la solution d'hydrogel sont des polymères réticulables lorsque soumis à un stimulus, tel qu'une température, un pH, des ions, etc. Avantageusement, la solution d'hydrogel utilisée est biocompatible, en ce sens qu'elle n'est pas toxique pour les cellules. Dans le cas où la composition d'intérêt à encapsuler comprend des cellules, la couche d'hydrogel permet avantageusement la diffusion d'oxygène et de nutriments, ainsi que celle de dioxyde de carbone et de déchets métaboliques pour alimenter les cellules et permettre leur survie. Les polymères de la solution d'hydrogel peuvent être d'origine naturelle ou synthétique. Par exemple, la solution d'hydrogel contient un ou plusieurs polymères parmi les polymères à base de sulfonate, tel que le polystyrène sulfonate de sodium, les polymères à base d'acrylate, tel que le polyacrylate de sodium, le polyéthylène glycol diacrylate, le composé gélatine méthacrylate, les polysaccharides, et notamment les polysaccharides d'origine bactérienne, telle que la gomme gellane, ou d'origine végétale, telles que la pectine ou l'alginate.

Dans un mode de réalisation, la solution d'hydrogel comprend au moins de l'alginate. Préférentiellement, la solution d'hydrogel ne comprend que de l'alginate. Dans le contexte de l'invention, on entend par « alginate » des polysaccharides linéaires formés à partir de β-D-mannuronate (M) et a-L-guluronate (G), des sels et dérivés de ceux-ci. Avantageusement, l'alginate est un alginate de sodium, composé à plus de 80% de G et moins de 20% de M, avec une masse moléculaire moyenne de 100 à 400 kDa (par exemple : PRONOVA® SLG100) et une à concentration totale comprise entre 0.5% et 5% en masse volumique (poids/volume).

Selon l'invention, l'aérosol de réticulation comprend au moins un agent réticulant adapté pour réticuler un hydrogel comprenant au moins un polymère hydrophile, tel que de l'alginate, lorsqu'il est mis en contact avec celui-ci. Préférentiellement, l'aérosol de réticulation est exempt de tensio-actif.

Dans un mode de réalisation, l'aérosol de réticulation est formé de microgouttes d'une solution de réticulation. Un tel aérosol peut être obtenu par tout moyen classiquement utilisé pour former un aérosol à partir d'une solution liquide, et notamment un brumisateur à membrane. Dans un autre mode de réalisation, l'aérosol de réticulation est constitué de microparticules solides d'agent de réticulation, par exemple sous forme de micro-poudre de carbonate et/ou de chlorure de calcium.

La solution de réticulation comprend avantageusement au moins un cation divalent. La solution de réticulation peut également être une solution comprenant un autre agent réticulant connu de l'alginate ou du polymère hydrophile à réticuler, ou un solvant, par exemple de l'eau ou un alcool, adapté pour permettre une réticulation par irradiation ou par toute autre technique connue dans l'art. Avantageusement, la solution de réticulation est une solution comprenant au moins un cation divalent. Préférentiellement, le cation divalent est un cation permettant de réticuler de l'alginate en solution. Il peut s'agir par exemple d'un cation divalent choisi dans le groupe comprenant Ca²⁺, Mg²⁺, Ba²⁺ et Sr²⁺, ou d'un mélange d'au moins deux de ces cations divalents. Le cation divalent, par exemple le Ca²⁺, peut être associé à un contre-ion pour former par exemple des solutions de type CaCl₂ ou CaCO₃, bien connues de l'homme du métier. La solution de réticulation peut également être une solution comprenant du CaCO₃ couplé à du Glucono delta-lactone (GDL) formant une solution de CaCO₃-GDL. La solution de réticulation peut également être un mélange de CaCO₃-CaSO₄-GDL. Dans un mode de mise en œuvre particulier du procédé selon l'invention, l'aérosol de réticulation est constitué de microgouttes d'une solution de réticulation comprenant du calcium, notamment sous la forme Ca²⁺. L'homme du métier est à même d'ajuster la nature du cation divalent et/ou du contre-ion, ainsi que sa concentration aux autres paramètres du procédé de la présente invention, notamment à la nature du polymère utilisé et à la vitesse et/ ou au degré de réticulation désiré(e). Par exemple, la concentration de cation divalent dans la solution de réticulation est comprise entre 10 et 1000 mM. La solution de réticulation peut comprendre d'autres constituants, bien connus de l'homme du métier, que ceux décrits ci-dessus, afin d'améliorer la réticulation de la gaine d'hydrogel dans les conditions, notamment de temps et/ou température, particulières.

Avantageusement, l'aérosol de réticulation est formé de microgouttes de solution de réticulation, dont un diamètre est avantageusement compris entre 0,5 et 20 µm, préférentiellement entre 1 et 10 µm, plus préférentiellement égal à 5 µm, +/- 2µm.

Dans un mode de réalisation, les microgouttes de solution de réticulation ou les microparticules solides formant l'aérosol ont une vitesse de chute inférieure à la vitesse à laquelle les gouttes mixtes sont projetées par le flux d'extrusion à travers l'aérosol de réticulation.

Préférentiellement, les microgouttes de solution de réticulation ou les microparticules solides formant l'aérosol sont en suspension statique dans l'air. Dans un mode de réalisation, les gouttes mixtes tombent par gravité à travers l'aérosol de réticulation et la vitesse de chute des gouttes mixtes à travers l'aérosol de réticulation est supérieure à la vitesse de chute des microgouttes ou les microparticules solides de réticulation.

Dans un mode de réalisation, l'aérosol de microgouttes de solution de réticulation présente une densité de 3*10³ microgouttes par cm³.

Avantageusement, le volume d'aérosol de microgouttes de solution de réticulation est maintenu dans une enceinte, dont les dimensions sont adaptées pour maintenir la densité d'aérosol et/ou la concentration souhaitées. Un tel confinement permet en outre de maintenir ledit aérosol stérile.

Les microgouttes de solution de réticulation viennent se coalescer, préférentiellement uniformément, à la surface des gouttes mixtes, de manière à favoriser la réticulation au moins partielle de la couche d'hydrogel sur toute la surface desdites gouttes mixtes. Par exemple, chaque goutte mixte de diamètre compris entre 50 et 500 µm est entourée d'au moins 100 microgouttes de solution de réticulation avant d'atteindre la surface de réception de gouttes mixtes au moins partiellement réticulées en capsules. L'homme du métier est à même d'adapter la hauteur de chute, la densité des microgouttes dans l'aérosol et/ou le débit d'aérosol, de manière à obtenir une couche de microgouttes autour de chaque goutte mixte en fonction de leur surface. La hauteur de chute s'entend de la distance parcourue par les gouttes mixtes à travers l'aérosol entre la sortie de l'extrudeuse et la surface de réception desdites gouttes mixtes au moins partiellement réticulées en capsules.

Dans un exemple de réalisation particulier, on utilise un aérosol de microgouttes de solution de réticulation ayant une densité de 3*10³ microgouttes par cm³, ledit aérosol étant pulvérisé avec un débit de 20L/heure, au sein d'une enceinte d'environ 20 cm de diamètre permettant de maintenir une densité d'aérosol suffisante en l'isolant de l'air ambiant, en ménageant une hauteur de chute des gouttes mixtes dans l'aérosol de 60 cm.

Dans un mode de réalisation particulier, les microgouttes de la solution de réticulation sont chargées, de sorte qu'elles se repoussent mutuellement, ce qui favorise l'électrodispersion des microgouttes dans l'aérosol. Pour cela, on peut par exemple appliquer un courant continu à la solution de réticulation avant pulvérisation ou nébulisation en aérosol.

De manière alternative ou complémentaire, les gouttes mixtes, et plus particulièrement la couche de solution d'hydrogel, sont chargées. Ainsi, les gouttes mixtes se dispersent sur un plus large rayon de section en traversant l'aérosol de réticulation. Ceci permet de réduire la hauteur de chute nécessaire en augmentant le volume d'aérosol utile à la réticulation. En effet une goutte mixte passant sur la même trajectoire que la précédente rencontre moins de microgouttes de réticulation. De plus, ceci réduit aussi les risques d'agglomération/fusion des gouttes mixtes en vol et à l'impact.

Dans le cas où les microgouttes de solution de réticulation et les gouttes mixtes sont chargées, les charges sont avantageusement opposées, de manière à favoriser le dépôt des microgouttes autour de la surface des gouttes mixtes. Selon l'invention, l'aérosol de réticulation a une osmolarité, ou concentration osmotique, de 0,3 à 3 fois supérieure à l'osmolarité de la composition d'intérêt formant le noyau central des capsules. Par exemple, l'aérosol de réticulation et la composition d'intérêt formant le noyau central des capsules peuvent être isoosmotiques, c'est-à-dire avoir des concentrations osmotiques sensiblement égales. Par exemple, dans le cas où la composition d'intérêt est une composition de cellules, l'aérosol de réticulation est formé à partir d'une solution de calcium à 100 mM. D'une manière générale, il faut éviter que l'aérosol de réticulation soit hyper-osmotique pour éviter la déformation de la goutte mixte générée par la pression osmotique et préserver la structure des capsules.

Selon l'invention, il est possible de réceptionner les capsules pré-rigidifiées après le passage dans l'aérosol de réticulation, dans un bain de récupération. Dans ce cas, le bain de récupération est préférentiellement isoosmotique avec la composition d'intérêt formant le noyau central des capsules.

Dans un mode de réalisation, le bain de récupération peut comprendre une solution de réticulation, afin de poursuivre au besoin la réticulation de l'hydrogel. Une telle solution de réticulation peut le cas échéant comporter un agent tensio-actif. Autrement, notamment dans le cas où la réticulation de l'hydrogel lors de la chute à travers l'aérosol de réticulation est suffisante, le bain de réticulation peut comporter une simple solution physiologique.

Avantageusement, les gouttes mixtes sont dépourvues d'agent tensioactif. Notamment, la composition d'hydrogel est avantageusement dépourvue de tensioactif. De même, les capsules selon l'invention sont avantageusement dépourvues de tensioactifs, au niveau de la couche d'hydrogel réticulé comme au niveau du noyau central.

Avantageusement, les solutions d'hydrogel et la composition d'intérêt sont coextrudées de manière à former des gouttes mixtes, et par la suite des capsules, sphériques de diamètre compris entre 50 et 500 µm. Dans un mode de réalisation particulier, l'épaisseur de l'enveloppe externe en hydrogel représente 5 à 40% du rayon des capsules. Dans le contexte de l'invention, « l'épaisseur » est la dimension s'étendant radialement par rapport au centre de la capsule. L'homme du métier sait adapter les paramètres de coextrusion pour obtenir des telles dimensions.

Selon l'invention, la composition d'intérêt s'entend de toute composition apte à être coextrudée avec une solution d'hydrogel pour former des capsules renfermant une goutte de ladite composition d'intérêt. Avantageusement, la composition d'intérêt est une composition liquide, aqueuse ou gélifiée. Dans un mode de réalisation, la composition aqueuse est une composition aqueuse gélifiée. Notamment, il est possible d'utiliser une composition comprenant des cellules, une composition alimentaire, une composition cosmétique ou une composition pharmaceutique. Par exemple, la composition d'intérêt est un gel de protéines et/ou un gel de cellules.

Selon l'invention, tout type de composition cellulaire peut être utilisé. Notamment, il est possible de réaliser des capsules d'hydrogel entourant des cellules souches pluripotentes humaines. Une cellule souche pluripotente, ou cellule pluripotente, s'entend d'une cellule qui a la capacité de former tous les tissus présents dans l'organisme d'origine entier, sans pour autant pouvoir former un organisme entier en tant que tel. Notamment, les capsules selon l'invention peuvent contenir des cellules souches induites à la pluripotence (IPS), par exemple à partir de cellules somatiques. Plus généralement, dans le contexte de l'invention, les compositions de cellules ne comprennent pas de cellules dérivées d'un embryon humain.

Dans un mode de réalisation particulier, la solution d'hydrogel est une solution d'alginate (par exemple le SLG100), la composition d'intérêt destinée à former le noyau central comprend des cellules (par exemple des cellules IPS resuspendues dans du Matrigel®), et l'aérosol de réticulation comprend des microgouttes d'une solution aqueuse de chlorure de calcium.

### EXEMPLE

### Exemple 1 : Protocole d'obtention de capsules d'hydrogel renfermant une composition de cellules humaines induites à la pluripotence.

### Solutions utilisées :

Solution 1, base de milieu DMEMF12 supplémentée avec 2µM Thiazovivin
Solution 2, PBS sans magnésium et sans calcium supplémenté avec 1µM Thiazovivin
Solution 3, tampon de détachement cellulaire non-enzymatique : RelesR™ supplémenté avec 2µM Thiazovivin.
Solution 4, milieu de culture cellules souches pluripotentes : MTeSR1™ hES/hIPS cell médium STEMCELL™).
Solution 4+, Solution 4 supplémentée avec 2µM Thiazovivin.
Solution 5, Matrigel™.
Solution 6, sorbitol 300mM avec 2µM Thiazovivin.

### Solution cellulaire :

Une boite de Pétri de cellules IPS humaines de 25cm² à 90% de confluence est utilisée par la suite pour correspondre aux volumes recommandés. Toutes les étapes suivantes sont effectuées à 4°C jusqu'à la sortie de l'extrudeur.
Étape 1 : Rincer les cellules avec la solution 1. Attendre de 10 minutes à 1 heure.
Étape 2 : Rincer deux fois avec 4 mL de solution 2.
Étape 3 : Aspirer délicatement la solution.
Étape 4 : Incuber les cellules avec 4 mL de solution 3 pendant 5-10 minutes.
Étape 5 : Détacher les cellules avec 2mL de solution 4+ avec une pipette à cône large pour réduire le stress de cisaillement.
Étape 6 : Centrifuger la suspension de cellules à 360 g pendant 5 minutes.
Étape 7 : Aspirer le surnageant.
Étape 8 : Resuspendre avec 0.5 mL de solution 4+.
Étape 9 : Centrifuger à nouveau à 360rcf et aspirer le surnageant.
Étape 10 : Re-suspendre le culot de cellules dans 70µL de solution 5 et 100µL de solution 6 (le volume du culot devrait-être de 30µL). La solution cellulaire est prête.

### Encapsulation :

Le dispositif d'encapsulation (extrudeuse) est préparé tel que décrit dans Alessandri et al., 2016 (« A 3D printed microfluidic device for production of functionalized hydrogel microcapsules for culture and differentiation of human Neuronal Stem Cells (hNSC) », Lab on a Chip, 2016, vol. 16, no. 9, p. 1593-1604), avec deux modifications : l'absence de surfactant dans la solution d'alginate et la présence d'une enceinte de 50cm de haut autour du bain de formation afin de contenir un aérosol issu de la nébulisation de la même solution que celle du bain de réticulation (CaCl2 300mosmol).

En résumé, les différentes parties du dispositif sont stérilisées (par autoclave) ; Les trois solutions nécessaires sont chargées sur trois pousses seringues, i) solution d'alginate (PRONOVA®SLG100 à 2% en masse dans de l'eau distillée), ii) solution intermédiaire (sorbitol à 300mM), iii) solution cellulaire (préparée à l'étape précédente) ; Les trois solutions sont co-injectées de manière concentrique grâce à un injecteur microfluidique qui permet de former un jet qui se fractionne en gouttes dont la couche extérieure est la solution d'alginate et le cœur la solution de cellules ;

Pour électro-disperser les gouttes, l'alginate a été chargé avec un courant continu à +2KV. Un anneau à la masse de 2cm de diamètre est disposé à 2mm de la pointe dans le plan perpendiculaire à l'axe du jet sortant de l'injecteur microfluidique pour générer le champ électrique.

Après fractionnement du jet et électro-dispersion des gouttes, celles-ci traversent l'aérosol de solution de réticulation contenue dans l'enceinte (de l'ordre de 3*10^{E}9 gouttelettes par mètre cube de gaz environ, chacune des gouttelettes a un diamètre de 1 à 10 µm et contient une solution aqueuse de calcium à 100mM). La coalescence de gouttelettes de solution de réticulation à la surface des gouttes mixtes rigidifie la surface de celles-ci et leur permet de pénétrer dans le bain de calcium (bain de récupération/réticulation) en conservant leur structure interne. Ces gouttes sont collectées dans un bain de calcium (à 100mM) qui finalise la réticulation de la solution d'alginate pour former la coque.

On note que dans ces conditions d'encapsulation, la réticulation de la couche d'alginate se fait spontanément.

### Traitement après encapsulation :

Étape 1 : Les capsules sont récupérées avec un tamis cellulaire 40µm puis après rinçage avec la solution 1 elles sont stockées dans une flasque de 75cm² avec 20mL de solution 4+.
Étape 2 : La flasque est gardée pendant 12h à l'incubateur à 37°C et 5% de CO².
Étape 3 : Changer le milieu pour la solution 4 pour permettre la formation des cystes.
Étape 4 : Après 24 à 72h des cystes de quelques dizaines de cellules se forment dans les capsules. Les microcompartiments cellulaires sont matures après 5 à 10 jours.

## Revendications

1. Procédé de fabrication d'une pluralité de capsules comprenant chacune une enveloppe externe d'hydrogel réticulée entourant un noyau central, selon lequel une solution d'hydrogel et une composition d'intérêt, destinée à former le noyau central, sont coextrudées de manière concentrique pour former des gouttes mixtes comprenant chacune une couche de solution d'hydrogel entourant une goutte de composition d'intérêt, l'étape de coextrusion étant réalisée au-dessus d'un aérosol de réticulation afin que les gouttes mixtes traversent ledit aérosol de réticulation, de sorte que la couche de solution d'hydrogel se réticule au moins partiellement autour de la goutte de composition d'intérêt au contact dudit aérosol, lesdites capsules étant sphériques et ayant un diamètre compris entre 50 et 500 µm, **caractérisé en ce que** la composition d'intérêt est une composition d'intérêt aqueuse et la concentration osmotique de l'aérosol de réticulation est comprise entre 0,3 à 3 fois la concentration osmotique de la composition d'intérêt formant le noyau central des capsules.

2. Procédé de fabrication de capsules selon la revendication 1, dans lequel l'aérosol de réticulation est formé de microgouttes de solution de réticulation, dont un diamètre est avantageusement compris entre 0,5 et 20 µm, préférentiellement entre 1 et 10 µm, préférentiellement égal à 5 µm, +/- 2µm.

3. Procédé de fabrication de capsules selon la revendication 2, dans lequel les microgouttes de solution de réticulation formant l'aérosol ont une vitesse de chute inférieure à la vitesse à laquelle les gouttes mixtes sont projetées par le flux d'extrusion à travers l'aérosol de réticulation.

4. Procédé de fabrication de capsules selon l'une des revendications précédentes, dans lequel les capsules sont plongées dans un bain de récupération après avoir traversé l'aérosol de réticulation, ledit bain de récupération comprenant avantageusement une solution de réticulation.

5. Procédé de fabrication de capsules selon l'une des revendications précédentes, selon lequel la goutte mixte est dépourvue d'agent tensioactif.

6. Procédé de fabrication de capsules selon l'une des revendications précédentes, selon lequel l'aérosol de réticulation et la composition d'intérêt formant le noyau central des capsules sont isoosmotiques.

7. Procédé de fabrication de capsules selon l'une des revendications précédentes, selon lequel les gouttes mixtes sont chargées de manière à se disperser et l'aérosol de réticulation est de charge opposée ou neutre.

8. Procédé de fabrication de capsules selon l'une des revendications précédentes, selon lequel la composition d'intérêt destinée à former le noyau central est choisie parmi une solution de cellules, une composition alimentaire, une composition cosmétique ou une composition pharmaceutique.

9. Procédé de fabrication de capsules selon l'une des revendications précédentes, selon lequel la solution d'hydrogel est une solution d'alginate, la composition d'intérêt destinée à former le noyau central comprend des cellules et l'aérosol de réticulation comprend des microgouttes d'une solution de chlorure de calcium.

## Patentansprüche

1. Verfahren zur Herstellung einer Vielzahl von Kapseln, umfassend jeweils eine äußere Hülle aus vernetztem Hydrogel, die einen zentralen Kern umgibt, wobei eine Hydrogel-Lösung und eine Zusammensetzung von Interesse, die den zentralen Kern bilden soll, konzentrisch koextrudiert werden, um gemischte Tropfen zu bilden, umfassend jeweils eine Schicht der Hydrogel-Lösung, die einen Tropfen der Zusammensetzung von Interesse umgibt, wobei der Schritt der Koextrusion über einem Vernetzungsaerosol durchgeführt wird, damit die gemischten Tropfen das Vernetzungsaerosol passieren, so dass die Schicht der Hydrogel-Lösung sich wenigstens partiell um den Tropfen der Zusammensetzung von Interesse bei Kontakt mit dem Aerosol vernetzt, wobei die Kapseln sphärisch sind und einen Durchmesser zwischen 50 und 500 µm besitzen, **dadurch gekennzeichnet, dass** die Zusammensetzung von Interesse eine wässrige Zusammensetzung von Interesse ist und die osmotische Konzentration des Vernetzungsaerosols zwischen dem 0,3 bis 3fachen der osmotischen Konzentration der Zusammensetzung von Interesse beträgt, die den zentralen Kern der Kapseln bildet.

2. Verfahren zur Herstellung von Kapseln gemäß Anspruch 1, wobei das Vernetzungsaerosol aus Mikrotropfen der Vernetzungslösung gebildet ist, deren Durchmesser vorteilhaft zwischen 0,5 und 20 µm, vorzugsweise zwischen 1 und 10 µm beträgt, vorzugsweise 5 µm ± 2 µm entspricht.

3. Verfahren zur Herstellung von Kapseln gemäß Anspruch 2, wobei die das Aerosol bildenden Mikrotropfen der Vernetzungslösung eine Fallgeschwindigkeit besitzen, die geringer ist als die Geschwindigkeit, mit der die gemischten Tropfen von dem Extrusionsstrom durch das Vernetzungsaerosol transportiert werden.

4. Verfahren zur Herstellung von Kapseln gemäß einem der vorhergehenden Ansprüche, wobei die Kapseln in ein Rückgewinnungsbad getaucht werden, nachdem sie das Vernetzungsaerosol passiert haben, wobei das Rückgewinnungsbad vorteilhaft eine Vernetzungslösung umfasst.

5. Verfahren zur Herstellung von Kapseln gemäß einem der vorhergehenden Ansprüche, wobei der gemischte Tropfen frei von Tensid ist.

6. Verfahren zur Herstellung von Kapseln gemäß einem der vorhergehenden Ansprüche, wobei das Vernetzungsaerosol und die Zusammensetzung von Interesse, die den zentralen Kern der Kapseln bildet, isoosmotisch sind.

7. Verfahren zur Herstellung von Kapseln gemäß einem der vorhergehenden Ansprüche, wobei die gemischten Tropfen geladen sind, um zu dispergieren, und das Vernetzungsaerosol eine entgegengesetzte Ladung trägt oder neutral ist.

8. Verfahren zur Herstellung von Kapseln gemäß einem der vorhergehenden Ansprüche, wobei die Zusammensetzung von Interesse, die den zentralen Kern bilden soll, aus einer Lösung von Zellen, einer Nahrungsmittelzusammensetzung, einer kosmetischen Zusammensetzung oder einer pharmazeutischen Zusammensetzung ausgewählt ist.

9. Verfahren zur Herstellung von Kapseln gemäß einem der vorhergehenden Ansprüche, wobei die Hydrogel-Lösung eine Alginat-Lösung ist, die Zusammensetzung von Interesse, die den zentralen Kern bilden soll, Zellen umfasst und das Vernetzungsaerosol Mikrotropfen einer Calciumchlorid-Lösung umfasst.

## Claims

1. A process for the manufacture of a plurality of capsules each comprising a crosslinked hydrogel outer shell surrounding a central core, wherein a hydrogel solution and an composition of interest, designed to form the central core, are concentrically coextruded to form mixed drops each comprising a layer of hydrogel solution surrounding a drop of composition of interest, the coextrusion step being carried out above a crosslinking aerosol so that the mixed drops pass through said crosslinking aerosol, so that the layer of hydrogel solution at least partially crosslinks around the drop of composition of interest in contact with said aerosol, said capsules being spherical and having a diameter comprised between 50 and 500 µm, **characterized in that** the composition of interest is an aqueous composition of interest and the osmotic concentration of the crosslinking aerosol is comprised between 0.3 to 3 times the osmotic concentration of the composition of interest forming the central core of the capsules.

2. The capsule manufacturing process as claimed in claim 1, wherein the crosslinking aerosol is formed from microdroplets of crosslinking solution, the diameter of which is advantageously comprised between 0.5 and 20 µm, preferentially between 1 and 10 µm, preferentially equal to 5 µm ± 2 µm.

3. The capsule manufacturing process as claimed in claim 2, wherein the microdroplets of crosslinking solution forming the aerosol have a falling velocity lower than the velocity at which the mixed drops are projected by the extrusion flow through the crosslinking aerosol.

4. The capsule manufacturing process as claimed in one of the preceding claims, wherein the capsules are immersed in a recovery bath after passing through the crosslinking aerosol, said recovery bath advantageously comprising a crosslinking solution.

5. The capsule manufacturing process as claimed in one of the preceding claims, wherein the mixed drop is free of surfactant.

6. The capsule manufacturing process as claimed in one of the preceding claims, wherein the crosslinking aerosol and the composition of interest forming the central core of the capsules are iso-osmotic.

7. The capsule manufacturing process as claimed in one of the preceding claims, wherein the mixed drops are charged so as to disperse and the crosslinking aerosol is oppositely charged or neutral.

8. The capsule manufacturing process as claimed in one of the preceding claims, wherein the composition of interest designed to form the central core is selected from a cell solution, a food composition, a cosmetic composition or a pharmaceutical composition.

9. The capsule manufacturing process as claimed in one of the preceding claims, wherein the hydrogel solution is an alginate solution, the composition of interest designed to form the central core comprises cells and the crosslinking aerosol comprises microdroplets of a calcium chloride solution.
